# EUROPEAN PATENT SPECIFICATION

(11) **EP 2 921 194 B1**
(45) Date of publication and mention of the grant of the patent: **08.06.2016**
(21) Application number: 15159469.4
(22) Date of filing: 17.03.2015
(51) Int. Cl.: A61M 25/00

(54) **Catheter**
Katheter
Cathéter

(30) Priority: 20.03.2014 JP 2014057791
(43) Date of publication of application: 23.09.2015
(73) Proprietor: ASAHI INTECC CO., LTD., Nagoya-shi, Aichi 463-0024 (JP)
(72) Inventor: Nihonmatsu, Masaaki c/o ASAHI INTECC CO., LTD., Nagoya-shi, Aichi 463-0024 (JP); Enami, Yukiko c/o ASAHI INTECC CO., LTD., Nagoya-shi, Aichi 463-0024 (JP)
(74) Representative: Winter, Brandl, Fürniss, Hübner, Röss, Kaiser, Polte - Partnerschaft mbB

(56) References cited:
- EP-A1- 2 692 388
- EP-A1- 2 695 635
- US-A1- 2001 027 310
- US-A1- 2011 071 503

## Description

### BACKGROUND OF THE INVENTION

### 1. Field of the Invention

The present invention relates to a catheter that is capable of easily changing a proceeding direction from a major canal (a main branch) to a minor canal (a side branch) at a bifurcation.

### 2. Description of the Related Art

When a stenosis site or an occlusion site is formed in a blood vessel, a bile duct, a pancreatic duct, or the like, flow of blood, bile (biliary fluid), pancreatic juice, or the like becomes weak. As a method of treating the stenosis site or the occlusion site, a treatment method using a catheter has been widely used.

Generally, a blood vessel, a bile duct, a pancreatic duct, or the like, increases the number of bifurcations of a major canal (a main branch) and a minor canal (a side branch) as the blood vessel, the bile duct, the pancreatic duct, or the like, approaches its end. Accordingly, when a stenosis site or an occlusion site is formed at an end portion of a blood vessel, a bile duct, a pancreatic duct, or the like, the catheter is required to have the ability to easily change its proceeding direction from a major canal (a main branch) to a minor canal (a side branch) at a bifurcation.

As a method of giving such an ability, there is known a catheter that is provided with a bend at a distal end of a catheter shaft in which the bend is bent obliquely towards a predetermined direction (see Japanese Patent Application Publication No. 2011-83596, for example). By providing such a bend, the distal end of the catheter can be oriented towards a direction that is offset with respect to a plane of the body portion of the catheter shaft.

### SUMMARY OF THE INVENTION

However, in the catheter of Japanese Patent Application Publication No. 2011-83596, since wire diameters of a first wire and a second wire that are woven together as a braid are the same, tensile strength of the first wire and the second wire are the same. Accordingly, even if a technician rotates the catheter in the right direction (clockwise) or in the left direction (anticlockwise), no difference is created in the rotational force (torque) transmitted to the distal end of the catheter (in other words, the rotational force (torque) is isotropic). With such a catheter, when the distal end of the catheter fails to enter a minor canal (a side branch) and is caught by a portion around the entrance of the minor canal (the side branch) at a bifurcation, even if the technician rotates the catheter in the right direction (clockwise) or in the left direction (anticlockwise), since the rotational force (torque) of the catheter is isotropic, the bend of the catheter shaft cannot be deformed. Accordingly, problems in that the distal end of the catheter cannot be inserted into the minor canal (the side branch) or changing the proceeding direction of the catheter from the major canal (the main branch) to the minor canal (minor branch) takes time are faced.

The present invention has been made in view of the above circumstances and aims to provide a catheter that can, at a bifurcation of a major canal (a main branch) and a minor canal (a side branch), easily change the proceeding direction from the major canal (the main branch) to the minor canal (the side branch) even when the distal end of the catheter is caught by a portion around the entrance of the minor canal (the side branch).

The above problems are overcome by the following device.

The present invention refers to a catheter including a catheter shaft having a proximal end and a distal end, and including a linear body portion extending from the proximal end of the catheter shaft and a bend that extends from a distal end of the body portion towards the distal end of the catheter shaft. In the catheter, a braid is embedded in the catheter shaft, the braid having a first wire that has a high tensile strength and a second wire that has a low tensile strength helically woven together. When viewed from a proximal end of the catheter shaft along the linear direction of the linear body portion, the bend forms an arc that extends in a winding direction of the first wire. I.e., in a projection plane (i.e, a plane perpendicular to the linear direction of the body portion) obtained by projecting the bend parallel to the linear direction of the linear body, the bend forms an arc that extends in a winding direction of the first wire.

The bend may extend not only in a two-dimensional plane perpendicular to the linear body portion (i.e., the projection plane) but also in a three-dimensional space defined by the projection plane and the linear direction of the body portion (i.e., the bend may also have an extension in the linear direction of the body portion). In this case, the bend extends helically and a handedness of the bend is the same as a handedness of the first wire, so that a stress applied to the bend during the operation of the catheter can be reduced.

Further, a handedness of the first wire is preferably different from a handedness of the second wire.

The number of strands constituting the first wire may be larger than the number of strands constituting the second wire, or a material of the first wire may have a higher tensile strength than a material of the second wire.

In one embodiment of the present invention, the bend includes, in a side view perpendicular to the body portion, a first tilted portion that extends in an oblique direction from the body portion through a first bend portion that is bent in a first direction, and a second tilted portion that is tilted from the first tilted portion through a second bend portion bent in a second direction and that is extended in a distal end direction, the second direction being a direction that is different from the first direction. In this embodiment, the helically extending bend may form a turn of at least 180°. Specifically, the helically extending bend may form a turn of at least 360°.

In another embodiment of the present invention, the second bend portion is bent in a sharp manner with respect to the first bend portion and forms an apex. In this embodiment, a helix angle of the helically extending bend may decrease towards the distal end of the catheter shaft.

In another embodiment of the present invention, the arc in the projection plane may extend elliptically.

According to the present invention, a braid is embedded in the catheter shaft, the braid having a first wire that has a high tensile strength and a second wire that has a low tensile strength woven together, and when viewed from the proximal end of the catheter shaft, the bend of the catheter shaft forms an arc that extends in a winding direction of the first wire. Regarding the tensile strength of the wires constituting the braid, since the tensile strength of the first wire is higher than the tensile strength of the second wire, when the technician rotates the catheter in the direction that is the same as the winding direction of the first wire, the braid can be functioned as a coil that is constituted by only the first wire while barely functioning the second wire. Accordingly, even when the distal end of the catheter is, at a bifurcation, caught by a portion around the entrance of the minor canal (the side branch), the technician rotating the catheter in the direction that is the same as the winding direction of the first wire can tighten the first wire functioning as a coil and deform the bend of the catheter shaft into a thin state and, accordingly, easily change the proceeding direction of the catheter from the major canal (the main branch) to the minor canal (the side branch). As a result, the amount of time taken to insert the catheter to a stenosis site or an occlusion site formed at the end portion is reduced.

### BRIEF DESCRIPTION OF THE DRAWINGS

Fig. 1 is a general view of a catheter of a first exemplary embodiment;
Fig. 2 is a diagram illustrating a catheter shaft, and note that illustrated is a state in which a portion of an outer layer and a portion of a braid are removed for convenience of description;
Fig. 3 is a cross-sectional view taken along line III-III of Fig. 2;
Fig. 4 is an enlarged diagram of portion IV of Fig. 1;
Fig. 5 is a diagram of the catheter shaft of Fig. 1 viewed from a proximal end side of the catheter shaft;
Fig. 6 illustrates a modification of the catheter of Fig. 1 and is a general view of a catheter of a second exemplary embodiment;
Fig. 7 is an enlarged diagram of portion VII of Fig. 6;
Fig. 8 is a diagram of the catheter shaft of Fig. 6 viewed from the proximal end side of the catheter shaft;
Fig. 9 is a diagram illustrating a modification of the catheter of Fig. 8;
Fig. 10 illustrates a modification of the catheter of Fig. 6 and is a general view of a catheter of a third exemplary embodiment;
Fig. 11 is an enlarged diagram of portion XI of Fig. 10;
Fig. 12 is a diagram of the catheter shaft of Fig. 10 viewed from the proximal end side of the catheter shaft; and
Figs. 13A to 13C are diagrams illustrating a manner in which the catheter changes the proceeding direction from a major canal (a main branch) to a minor canal (a side branch).

### DESCRIPTION OF THE PREFERRED EMBODIMENTS

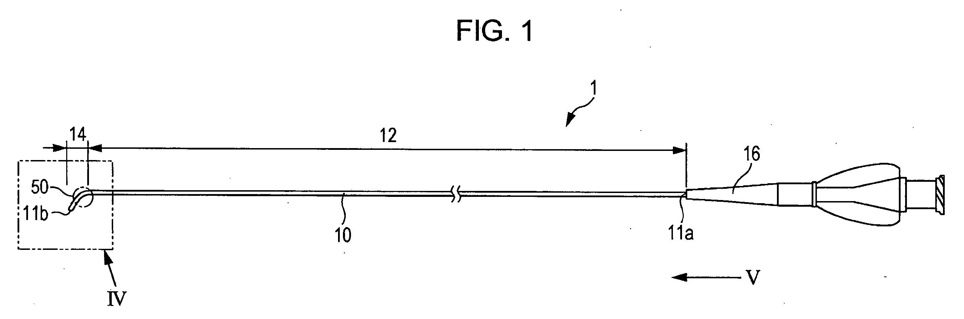

Referring to Figs. 1 to 5, the present invention will be described with an exemplary case in which a catheter 1 of the present exemplary embodiment is used. In Figs. 1, 2 and 4, the left side of the drawing is a distal end side (a distal side) of the catheter 1 which is inserted into the body and the right side of the drawing is a proximal end side (a proximal side or a base end side) of the catheter 1 on which manipulation is carried out by a technician, such as a doctor.

The catheter 1 is used to treat, for example, a stenosis site or an occlusion site that is formed in a blood vessel, a bile duct, a pancreatic duct, or the like. As illustrated in Fig. 1, the catheter 1 mainly includes a catheter shaft 10 including a proximal end 11a, a linear body portion 12, a bend 14, and a distal end 11b, and a connector 16 attached to the proximal end 11a of the catheter shaft 10.

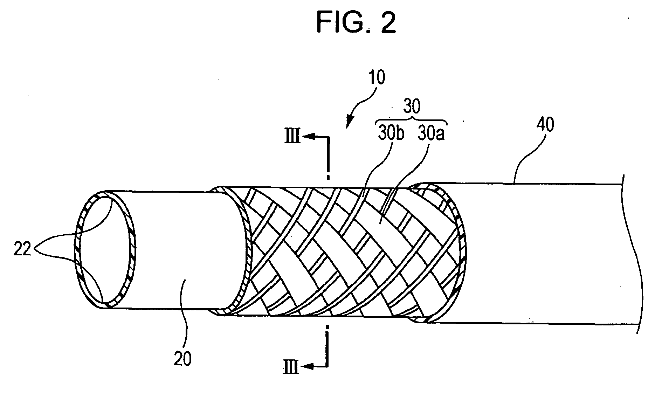

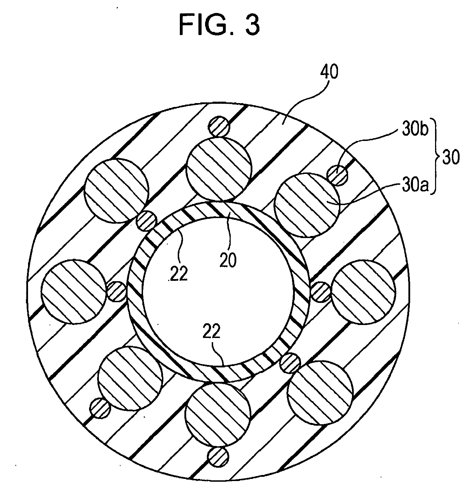

As illustrated in Figs. 2 and 3, the catheter shaft 10 includes, in a radial direction and in order from the inner side, an inner layer 20, a braid 30 that is a reinforcement body, and an outer layer 40. Note that although Fig. 2 is an illustration of the catheter shaft 10, a portion of the braid 30 and a portion of the outer layer 40 have been removed for convenience of description. Fig. 3 is a cross-sectional view taken along line III-III of Fig. 2.

The inner layer 20 is formed of resin and includes a lumen 22 for inserting a guide wire or another catheter therein. The resin material forming the inner layer 20 is not limited to a particular material; however, when considering the sliding property of the guide wire or the catheter that is inserted inside the inner layer 20, the resin material is desirably polytetrafluoroethylene (PTFE).

The braid 30 that is a reinforcement body is formed on the outer periphery of the inner layer 20. The braid 30 is a member in which a first wire 30a that has a high tensile strength and a second wire 30b that has a low tensile strength are helically woven into a net-like form (a mesh-like form). When viewed in a V-direction (the linear direction of the linear body portion 12) of Fig. 1 from the proximal end 11a of the catheter shaft 10, the first wire 30a that has a high tensile strength is wound in the right-handed direction (clockwise) and the second wire 30b that has a low tensile strength is wound in the left-handed direction (anticlockwise), and the first wire 30a and the second wire 30b are alternately woven together (see Fig. 2). Accordingly, the handedness of the first wire 30a is different from (opposite to) the handedness of the second wire 30b.

The materials of the first wire 30a and the second wire 30b constituting the braid 30 may be the same, or different materials may be used. In either case, the tensile strength of the first wire 30a needs to be higher than the tensile strength of the second wire 30b. In the present exemplary embodiment, the same metal material (stainless steel (SUS316)) is used in the first wire 30a and the second wire 30b, and the wire diameter of the first wire 30a is large and the wire diameter of the second wire 30b is small (see Fig. 3). However, the method of making the tensile strength of the first wire 30a higher than the tensile strength of the second wire 30b is not limited to the above method. For example, when the first wire 30a and the second wire 30b are formed of the same material, the number of wires constituting the first wire 30a may be set large so that the tensile strength is high and the number of wires constituting the second wire 30b may be set small so that the tensile strength is low. Furthermore, when the first wire 30a and the second wire 30b are formed with the same wire diameter, a material with a high tensile strength (tungsten, for example) may be used in the first wire 30a for high tensile strength and the material with a low tensile strength (stainless steel (SUS316), for example) may be used in the second wire 30b for low tensile strength. Note that the materials of the first wire 30a and the second wire 30b are not limited to metal and, for example, carbon fiber or reinforced fiberglass may be used.

Furthermore, in the present exemplary embodiment, although the cross-sectional shapes of the first wire 30a and the second wire 30b are both round (see Fig. 3), the cross-sectional shapes are not limited to the above shapes. For example, the cross-sectional shape of the first wire 30a may be rectangular and the cross-sectional shape of the second wire 30b may be round.

The outer layer 40 formed of resin that is formed on the outer periphery of the braid 30 covers the inner layer 20 and the braid 30. The resin material forming the outer layer 40 is not limited to a particular material and may be, for example, polyamide, polyamide elastomer, polyester, or polyurethane.

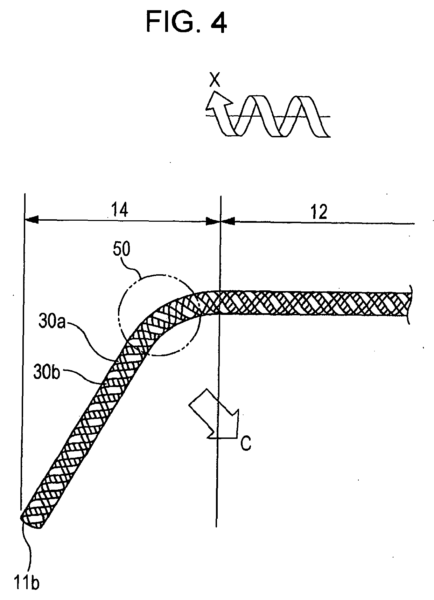

Although Fig. 4 is an enlarged drawing of portion IV of Fig. 1, for convenience of description, the outer layer 40 that covers the catheter shaft 10 is removed such that Fig. 4 illustrates the braid 30 in which the first wire 30a and the second wire 30b are woven together. As illustrated in Fig. 4, the bend 14 extends from the body portion 12 to the distal end 11b and is tilted from the body portion 12 through the first bend portion 50 that is bent in a first direction C. A winding direction X of the first wire 30a embedded in the catheter shaft 10 is the right-handed direction (clockwise).

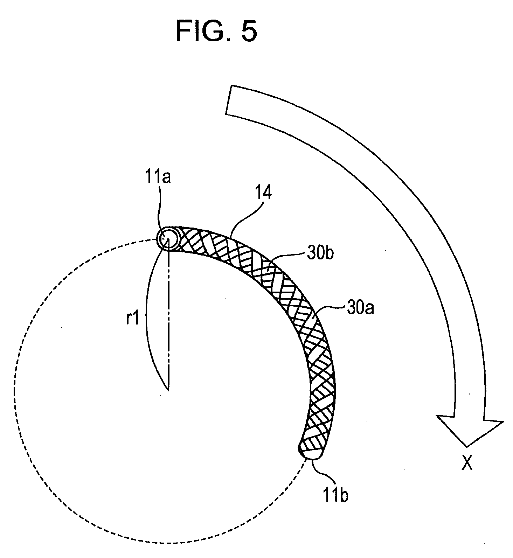

Fig. 5 is a diagram of the catheter shaft 10 when viewed from the proximal end 11a of the catheter shaft 10 in the V-direction of Fig. 1. I.e., Fig. 5 shows a parallel projection obtained by projecting the bend 14 parallel to the V-direction of Fig. 1. The diagram of Fig.5 represents a projection plane perpendicular to the linear direction of the body portion Similar to Fig. 4, for convenience of description, the outer layer 40 that covers the catheter shaft 10 is removed such that the braid 30 in which the first wire 30a and the second wire 30b are woven together is illustrated. As illustrated in Fig. 5, when viewed in the V-direction of Fig. 1 from the proximal end 11a of the catheter shaft 10, the bend 14 forms an arc with a radius of r1 extending in the right-handed direction (clockwise) that is the winding direction X of the first wire 30a that has a high tensile strength. In other words, when viewed in the V-direction of Fig. 1 from the proximal end 11a of the catheter shaft 10, the portion from the proximal end 11a to the distal end 11b of the catheter shaft 10 is on a circumference with a radius of r1. I.e., the bend 14 extends helically and a handedness of the bend is the same as a handedness of the first wire 30a.

As described above, in the catheter 1, regarding the tensile strength of the wires constituting the braid 30, the tensile strength of the first wire 30a is higher than the tensile strength of the second wire 30b. When the technician rotates the catheter 1 in the right-handed direction (clockwise) that is the same direction as the winding direction X of the first wire 30a, the braid 30 can be functioned as a coil that is constituted by only the first wire 30a while barely functioning the second wire 30b. Accordingly, even when the distal end of the catheter 1 is, at a bifurcation, caught by a portion around the entrance of a minor canal (a side branch), the technician rotating the catheter 1 in the right-handed direction (clockwise), which is the same direction as the winding direction X of the first wire 30a, can tighten the first wire 30a functioning as a coil and deform the bend 14 of the catheter shaft 10 into a thin state and, accordingly, easily change the proceeding direction of the catheter 1 from a major canal (a main branch) to the minor canal (the side branch). As a result, the amount of time taken to insert the catheter 1 to a stenosis site or an occlusion site formed at an end portion can be reduced.

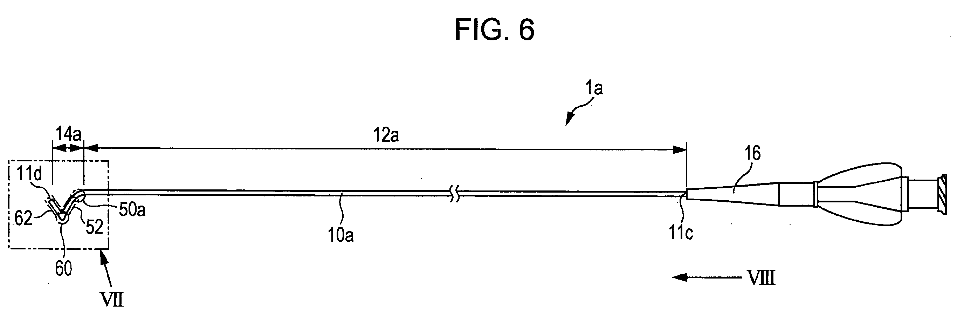

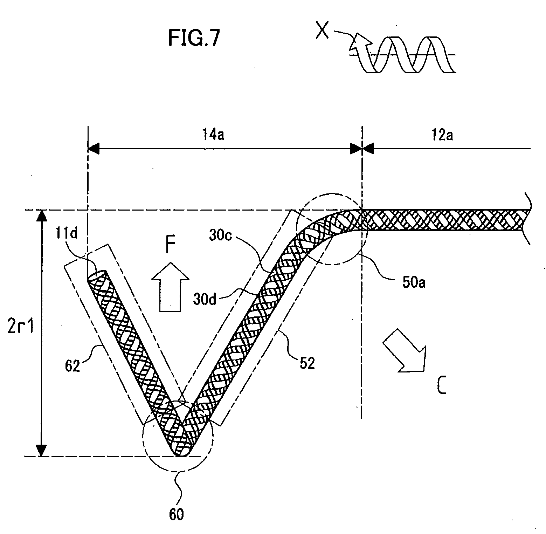

Referring next to Figs. 6 to 9, a catheter 1a of a second exemplary embodiment will be described. Note that similar to Figs. 1, 2, and 4, in Figs. 6 and 7, the left side of the drawing is a distal end side (a distal side) of the catheter 1a which is inserted into the body and the right side of the drawing is a proximal end side (a proximal side or a base end side) of the catheter 1a on which manipulation is carried out by a technician, such as a doctor.

Only the points different from the catheter 1 illustrated in Figs. 1 to 5 will be described. As illustrated in Fig. 6 and Fig. 7 that is an enlarged drawing of a portion VII of Fig. 6, in the catheter 1a, a bend 14a that extends from the body portion 12a towards the distal end 11d includes a first tilted portion 52 that extends in an oblique direction from the body portion 12a through the first bend portion 50a that is bent in a first direction C, and a second tilted portion 62 that is tilted from the first tilted portion 52 and that extends in the distal end direction through a second bend portion 60 that is bent in a second direction F that is a direction different from the first direction C. In other words, the bend 14a forms a turn of at least 180°. Similar to Fig. 4, the winding direction X of a first wire 30c embedded in the catheter shaft 10a is the right-handed direction (clockwise). Meanwhile, a second wire 30d that is embedded in the catheter shaft 10a is wound in the left-handed direction (anticlockwise).

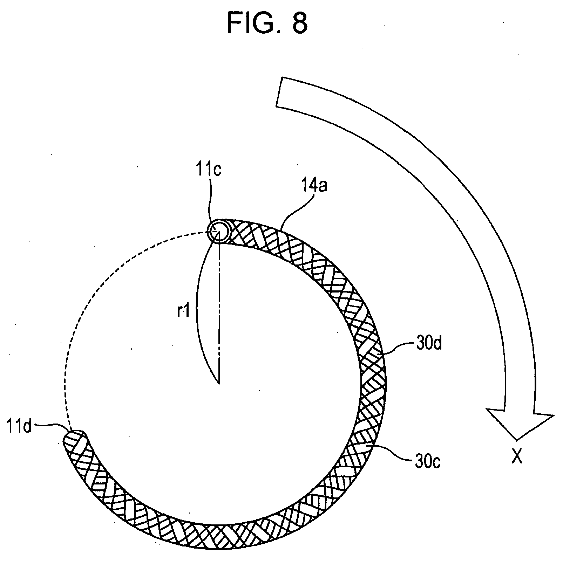

Fig. 8 is a diagram (parallel projection) of the catheter shaft 10a when viewed from a proximal end 11c of the catheter shaft 10a in the VIII-direction (the linear direction of the linear body portion 12a) of Fig. 6. As illustrated in Fig. 8, when viewed in the VIII-direction of Fig. 6 from the proximal end 11c of the catheter shaft 10a, the bend 14a forms an arc with a radius of r1 extending in the right-handed direction (clockwise) that is the winding direction X of the first wire 30c that has a high tensile strength. In other words, when viewed in the VIII-direction of Fig. 6 from the proximal end 11c of the catheter shaft 10a, the portion from the proximal end 11c to a distal end 11d of the catheter shaft 10a is on a circumference with a radius of r1.

Compared with the catheter 1, in the catheter 1a, the length of the first wire 30c that functions as a coil in the bend 14a is longer (see Figs. 5 and 8). In the bend 14a, since the first wire 30c that functions as a coil is long, the bend 14a can be easily deformed into a thin state when the technician rotates the catheter 1a in the right-handed direction (clockwise), which is the same direction as the winding direction X of the first wire 30c. Furthermore, since the second tilted portion 62 extends in the distal end direction, when the distal end of the catheter 1a is, at the bifurcation, caught by a portion around the entrance of the minor canal (the side branch), the technician rotating the catheter 1a in the right-handed direction (clockwise), which is the same direction as the winding direction X of the first wire 30c, and pushing the distal end of the catheter 1a at the same time can transmit the pushing force of the technician to the distal end of the catheter 1a while the bend 14a is deformed in a thin state. Accordingly, the catheter 1a can be easily guided into the minor canal (the side branch) without the distal end of the catheter 1a being caught again by a portion around the entrance of the minor canal (the side branch).

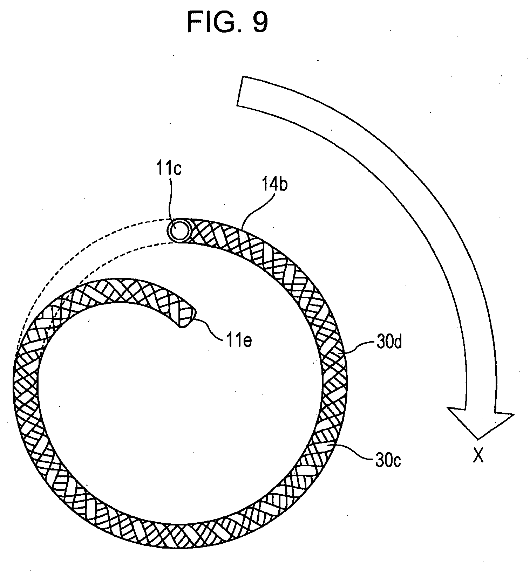

Note that as a modification of the catheter 1a of Fig. 8, as illustrated in Fig. 9, as a bend 14b of the catheter shaft 10a, an arc that is longer than a circumference with a radius of r1 (longer than a single lap of the circumference) may be formed in the right-handed direction (clockwise), which is the winding direction X of the first wire 30c that has a high tensile strength, when viewed in the VIII-direction of Fig. 6 from the proximal end 11c of the catheter shaft 10a. In other words, the bend 14b forms a turn of at least 360°. With the above, the length of the first wire 30c that functions as a coil in the bend 14b can be extended further. Note that in Fig. 9, in order to facilitate understanding, a distal end 11e of the catheter shaft 10a is illustrated at a displaced position with respect to the circumference with a radius of r1, however the position of the distal end 11e of the catheter shaft 10a is not limited to the above position and, similar to that of Fig. 8, the distal end 11e of the catheter shaft 10a may be positioned on the circumference with a radius of r1.

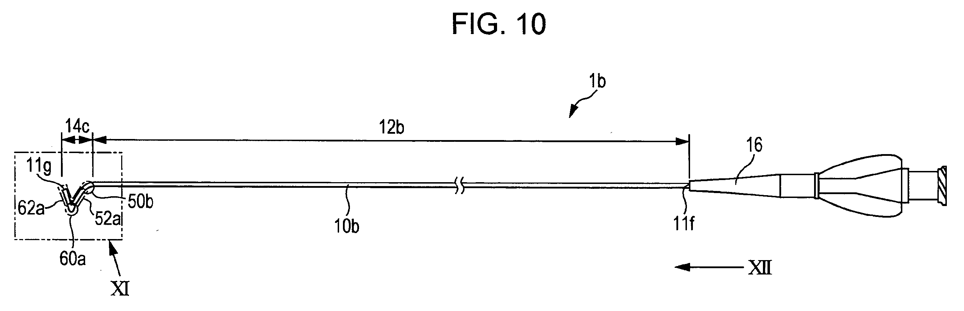

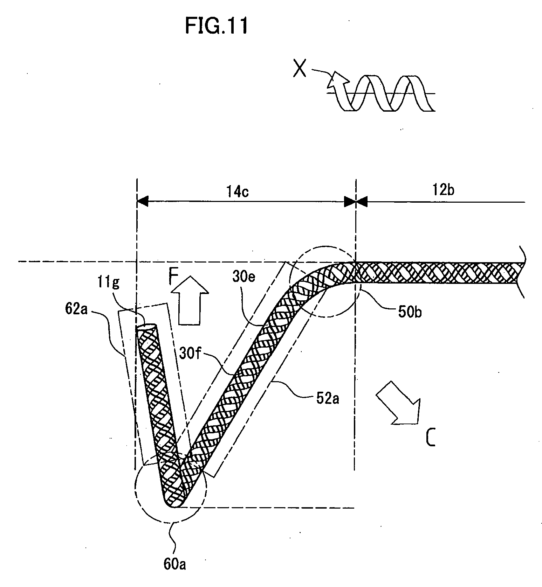

Referring next to Figs. 10 to 12, a catheter 1b of a third exemplary embodiment will be described. Note that similar to Figs. 1, 2, 4, 6, and 7, in Figs. 10 and 11, the left side of the drawing is a distal end side (a distal side) of the catheter 1b which is inserted into the body and the right side of the drawing is a proximal end side (a proximal side or a base end side) of the catheter 1b on which manipulation is carried out by the technician, such as a doctor.

Only the points different from the catheter 1a illustrated in Figs. 6 to 9 will be described. As illustrated in Fig. 10 and Fig. 11 that is an enlarged drawing of a portion XI of Fig. 10, in the catheter 1b, a bend 14c that extends from the body portion 12b towards the distal end 11g includes a first tilted portion 52a that extends in an oblique direction from the body portion 12b through a first bend portion 50b that is bent in the first direction C, and a second tilted portion 62a that is tilted from the first tilted portion 52a and that extends in the distal end direction through a second bend portion 60a that is bent in a second direction F that is a direction different from the first direction C. Similar to Fig. 7, the winding direction X of a first wire 30e embedded in the catheter shaft 10b is the right-handed direction (clockwise). Meanwhile, a second wire 30f that is embedded in the catheter shaft 10b is wound in the left-handed direction (anticlockwise).

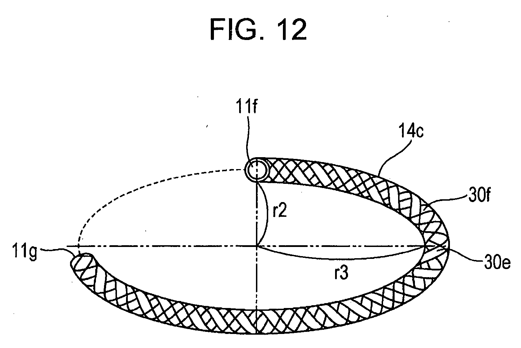

Fig. 12 is a diagram (parallel projection) of the catheter, shaft 10b when viewed from a proximal end 11f of the catheter shaft 10b in the XII-direction (the linear direction of the linear body portion 12b) of Fig. 10. As illustrated in Fig. 12, when viewed in the XII-direction of Fig. 10 from the proximal end 11f of the catheter shaft 10b, the bend 14c forms an arc extending in the right-handed direction (clockwise) that is the winding direction X of the first wire 30e that has a high tensile strength, however, different from Fig. 8, the arc is an ellipse with a minor axis of r2. In other words, when viewed in the XII-direction of Fig. 10 from the proximal end 11f of the catheter shaft 10b, the portion from the proximal end 11f to a distal end 11g of the catheter shaft 10b is on a circumference of the ellipse with the minor axis of r2 and a major axis of r3.

Compared with the catheter 1a, in the catheter 1b, the second bend portion 60a is bent in a sharp manner with respect to the first bend portion 50b and forms an apex. In other word, a helix angle of the bend 14c decreases towards the distal end 11g. Accordingly, the apex of the second bend portion 60a can be abutted against the wall of the major canal (the main branch) and a strong backup force can be created with the bend 14c of the catheter shaft 10b; accordingly, the rotational force (torque) of the technician can be transmitted to the distal end of the catheter 1b in an easier manner. As a result, the proceeding direction of the catheter 1b can be changed from the major canal (the main branch) to the minor canal (the side branch) in an easier manner.

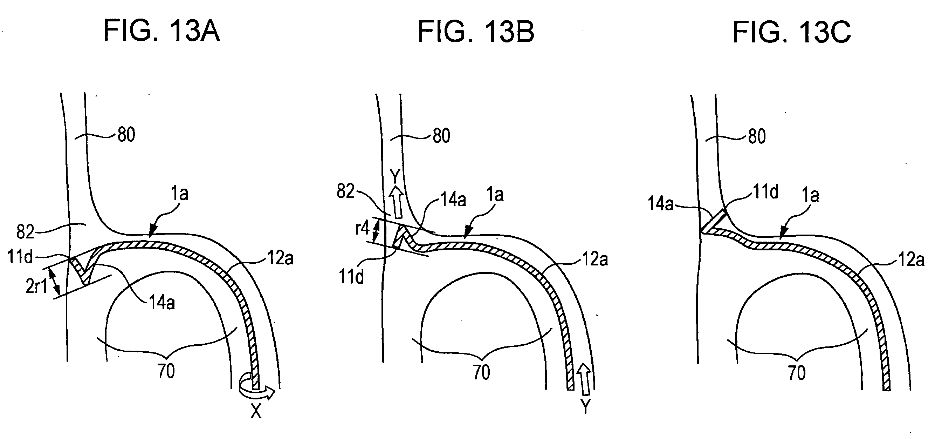

Referring next to Figs. 13A to 13C, a manner in which the catheter 1, 1a, or 1b changes the proceeding direction from a major canal (a main branch) 70 to a minor canal (a side branch) 80 will be described. For convenience of description, the catheter 1a is used in Figs. 13A to 13C; however, the same can be applied to the catheters 1 and 1b.

Fig. 13A is a diagram that illustrates a state in which the distal end of the catheter 1a is caught by a portion around an entrance 82 of the minor canal (the side branch) 80. The bend 14a of the catheter shaft 10a forms the arc with a radius of r1 extending in the right-handed direction (clockwise) that is the winding direction X of the first wire 30c that has a high tensile strength. At this time, the width of the bend 14a is r1 x 2 = 2r1 at the most (see Fig. 7). Fig. 13B is a diagram that illustrates a state in which the technician has rotated the catheter 1a in the right-handed direction (clockwise) that is the same direction as the winding direction X of the first wire 30c. In the bend 14a of the catheter shaft 10a, the first wire 30c functions as a coil and rotates while being tightened so as to become slightly thinner. Accordingly, the distal end of the catheter 1a that had been caught is moved away from the portion around the entrance 82 of the minor canal (the side branch) 80. At this time, the width of the bend 14a is r4 that is smaller than 2r1 (r4 < 2r1). In the above state, when the technician pushes the catheter 1a in a distal end direction Y, as illustrated in Fig. 13C, the proceeding direction can be changed from the major canal (the main branch) 70 to the minor canal (the side branch) 80 without the distal end of the catheter 1a being caught again by a portion around the entrance 82 of the minor canal (the side branch) 80.

Note that in the above description, although the winding direction X of the first wires 30a, 30c, and 30e constituting the braid 30 of the catheters 1, 1a, and 1b, respectively, is the right-handed direction (clockwise), the winding direction X is not limited to the above. When the winding direction of the first wires 30a, 30c, and 30e are to be the left-handed direction (anticlockwise), the bends 14, 14a, 14b, and 14c of the catheter shafts 10, 10a, and 10b may be made to form an arc extending in the left-handed direction (anticlockwise) when the bends 14, 14a, 14b, and 14c of the catheter shafts 10, 10a, and 10b are viewed in the V-direction of Fig. 1, the VIII-direction of the Fig. 6, and in the XII-direction of the Fig. 10 from the proximal ends 11a, 11c, and 11e of the catheter shafts 10, 10a, and 10b.

As described above, in the catheter 1, 1a, and 1b, regarding the tensile strength of the wires constituting the braid 30, the tensile strength of the first wire 30a, 30c, and 30e are higher than the tensile strength of the second wire 30b, 30d, and 30f, respectively. Accordingly, even when the distal end of the catheter 1, 1a, or 1b is, at the bifurcation, caught by a portion around the entrance 82 of the minor canal (the side branch) 80, the technician rotating the catheter 1, 1a, or 1b in the direction that is the same as the winding direction X of the first wire 30a, 30c, or 30e can tighten the first wire 30a, 30c, or 30e functioning as a coil and deform the bend 14, 14a, or 14c of the catheter shaft 10, 10a, or 10b into a thin state and, accordingly, easily change the proceeding direction of the catheter 1, 1a, or 1b from the major canal (the main branch) 70 to the minor canal (the side branch) 80. As a result, the amount of time taken to insert the catheter 1, 1a, or 1b to a stenosis site or an occlusion site formed at the end portion can be reduced.

## Claims

1. A catheter (1,1a,1b), comprising:
a catheter shaft (10a,10b,10c) having a proximal end (11a,11c,11f) and a distal end (11b, 11d, 11g), and including
a linear body portion (12,12a,12b) extending from the proximal end (11a,11c,11f), and
a bend (14,14a,14b,14c) that extends from the body portion towards the distal end (11b, 11d, 11g), wherein
a braid (30) is installed inside the catheter shaft, **characterized in that**
the braid (30) comprises a first wire (30a,30c,30e) that has a high tensile strength and a second wire (30b,30d,30f) that has a low tensile strength, which are helically woven together, and
when viewed from a proximal end (11a,11c,11f) of the catheter shaft along the linear direction of the linear body portion (12,12a,12b), the bend forms an arc that extends in a winding direction (X) of the first wire.

2. The catheter (1,1a,1b) according to Claim 1, wherein
a handedness of the first wire (30a,30c,30e) is different from a handedness of the second wire (30b,30d,30f).

3. The catheter (1,1a,1b) according to Claim 1 or 2, wherein
the number of strands constituting the first wire (30a,30c,30e) is larger than the number of strands constituting the second wire (30b,30d,30f).

4. The catheter (1,1a,1b) according to any one of Claims 1 to 3, wherein
a material of the first wire (30a,30c,30e) has a higher tensile strength than a material of the second wire (30b,30d,30f).

5. The catheter (1a,1b) according to any one of Claims 1 to 4, wherein
in a side view perpendicular to the linear body portion (12a,12b), the bend includes
a first tilted portion (52,52a) that extends in an oblique direction from the body portion through a first bend portion (50a,50b) that is bent in a first direction (C)
a second tilted portion (62,62a) that is tilted from the first tilted portion through a second bend portion (60,60a) bent in a second direction (F) and that is extended in a distal end direction, the second direction being a direction that is different from the first direction.

6. The catheter (1b) according to Claim 5, wherein
the second bend portion (60a) is bent in a sharp manner with respect to the first bend portion (50b) and forms an apex.

7. The catheter (1,1a,1b) according to any one of Claims 1 to 6, wherein
the bend extends helically and a handedness of the bend is the same as a handedness of the first wire (30a,30c,30e).

8. The catheter (1a,1b) according to Claim 7, wherein
the helically extending bend forms a turn of at least 180°.

9. The catheter (1a,1b) according to Claim 7, wherein
the helically extending bend forms a turn of at least 360°.

10. The catheter (1b) according to Claim 8 or 9, wherein
a helix angle of the helically extending bend decreases towards the distal end (11g).

11. The catheter (1b) according to any one of Claims 8 to 10, wherein
the arc extends elliptically.

## Patentansprüche

1. Katheter (1,1a,1b), mit:
einem Katheterschaft (10a,10b,10c), der ein proximales Ende (11a,11c,11f) und ein distales Ende (11b, 11d, 11g) hat und aufweist
einen linearen Körperabschnitt (12,12a,12b), der sich von dem proximalen Ende (11a,11c,11f) aus erstreckt, und
einen Krümmungsabschnitt (14,14a,14b,14c), der sich von dem Körperabschnitt aus in Richtung des distalen Endes (11b, 11d, 11g) erstreckt, wobei
im Inneren des Katheterschafts ein Geflecht (30) angeordnet ist, **dadurch gekennzeichnet, dass**
das Geflecht (30) einen ersten Draht (30a,30c,30e) mit einer hohen Zugfestigkeit und einen zweiten Draht (30b,30d,30f) mit einer niedrigen Zugfestigkeit aufweist, die schraubenförmig miteinander verwebt sind, und
der Krümmungsabschnitt, von dem proximalen Ende (11a,11c,11f) des Katheterschafts aus in linearer Richtung des linearen Körperabschnitts (12,12a,12b) gesehen, einen Bogen formt, der sich in der Wicklungsrichtung (X) des ersten Drahts erstreckt.

2. Katheter (1,1a,1b) nach Anspruch 1, wobei
die Gangrichtung des ersten Drahts (30a,30c,30e) von der Gangrichtung des zweiten Drahts (30b,30d,30f) verschieden ist.

3. Katheter (1,1a,1b) nach Anspruch 1 oder 2, wobei
die Anzahl der Litzendrähte, die den ersten Draht (30a,30c,30e) bilden, größer ist als die Anzahl der Litzendrähte, die den zweiten Draht (30b,30d,30f) bilden.

4. Katheter (1,1a,1b) nach einem der Ansprüche 1 bis 3, wobei
das Material des ersten Drahts (30a,30c,30e) eine höhere Zugfestigkeit hat als das Material des zweiten Drahts (30b,30d,30f).

5. Katheter (1a,1b) nach einem der Ansprüche 1 bis 4, wobei
der Krümmungsabschnitt, in einer Seitenansicht senkrecht zu dem linearen Körperabschnitt (12a,12b) gesehen, aufweist
einen ersten schiefwinkligen Abschnitt (52,52a), der sich durch einen ersten Biegeabschnitt (50a,50b), der in eine erste Richtung (C) gebogen ist, in einem schiefen Winkel zum Körperabschnitt erstreckt, und
einen zweiten schiefwinkligen Abschnitt (62,62a), der durch einen zweiten Biegeabschnitt (60,60a), der in eine von der ersten Richtung verschiedene zweite Richtung (F) gebogen ist, in einem schiefen Winkel zum ersten schiefwinkligen Abschnitt angeordnet ist und sich in Richtung des distalen Endes erstreckt.

6. Katheter (1b) nach Anspruch 5, wobei
der zweite Biegeabschnitt (60a) gegenüber dem ersten Biegeabschnitt (50b) stark gebogen ist und einen Scheitelpunkt bildet.

7. Katheter (1,1a,1b) nach einem der Ansprüche 1 bis 6, wobei
sich der Krümmungsabschnitt schraubenförmig erstreckt, und die Gangrichtung des Krümmungsabschnitts der Gangrichtung des ersten Drahts (30a,30c,30e) entspricht.

8. Katheter (1a,1b) nach Anspruch 7, wobei
der sich schraubenförmig erstreckende Krümmungsabschnitt eine Windung von wenigstens 180° formt.

9. Katheter (1a,1b) nach Anspruch 7, wobei
der sich schraubenförmig erstreckende Krümmungsabschnitt eine Windung von wenigstens 360° formt.

10. Katheter (1b) nach Anspruch 8 oder 9, wobei
der Steigungswinkel des sich schraubenförmig erstreckenden Krümmungsabschnitts zum distalen Ende (11g) hin abnimmt.

11. Katheter (1b) nach einem der Ansprüche 8 bis 10, wobei
sich der Bogen elliptisch erstreckt.

## Revendications

1. Cathéter (1, 1a, 1b), comprenant :
une tige (10a, 10b, 10c) de cathéter ayant une extrémité proximale (11a, 11c, 11f) et une extrémité distale (11b, 11d, 11g), et incluant
une partie corps linéaire (12, 12a, 12b) s'étendant depuis l'extrémité proximale (11a, 11c, 11f), et
un coude (14, 14a, 14b, 14c) qui s'étend depuis la partie corps vers l'extrémité distale (11b, 11d, 11g), dans lequel
une tresse (30) est installée à l'intérieur de la tige de cathéter, **caractérisé en ce que**
la tresse (30) comprend un premier fil (30a, 30c, 30e) qui présente une résistance à la traction élevée et un deuxième fil (30b, 30d, 30f) qui présente une résistance à la traction faible, qui sont tissés ensemble de manière hélicoïdale, et
quand on regarde depuis une extrémité proximale (11a, 11c, 11f) de la tige de cathéter le long de la direction linéaire de la partie corps linéaire (12, 12a, 12b), le coude forme un arc qui s'étend dans une direction d'enroulement (X) du premier fil.

2. Cathéter (1, 1a, 1b) selon la revendication 1, dans lequel
un sens de rotation du premier fil (30a, 30c, 30e) est différent d'un sens de rotation du deuxième fil (30b, 30d, 30f).

3. Cathéter (1, 1a, 1b) selon la revendication 1 ou 2, dans lequel
le nombre de brins constituant le premier fil (30a, 30c, 30e) est supérieur au nombre de brins constituant le deuxième fil (30b, 30d, 30f).

4. Cathéter (1, 1a, 1b) selon l'une quelconque des revendications 1 à 3, dans lequel
un matériau du premier fil (30a, 30c, 30e) présente une résistance à la traction plus élevée qu'un matériau du deuxième fil (30b, 30d, 30f).

5. Cathéter (1a, 1b) selon l'une quelconque des revendications 1 à 4, dans lequel
sur une vue latérale perpendiculaire à la partie corps linéaire (12a, 12b), le coude inclut
une première partie inclinée (52, 52a) qui s'étend dans une direction oblique depuis la partie corps à travers une première partie de coude (50a, 50b) qui est coudée dans une première direction (C)
une deuxième partie inclinée (62, 62a) qui est inclinée depuis la première partie inclinée à travers une deuxième partie de coude (60, 60a) coudée dans une deuxième direction (F) et qui est étendue dans une direction d'extrémité distale, la deuxième direction étant une direction qui est différente de la première direction.

6. Cathéter (1b) selon la revendication 5, dans lequel
la deuxième partie de coude (60a) est coudée de manière aiguë par rapport à la première partie de coude (50b) et forme un sommet.

7. Cathéter (1, 1a, 1b) selon l'une quelconque des revendications 1 à 6, dans lequel
le coude s'étend de manière hélicoïdale et un sens de rotation du coude est le même qu'un sens de rotation du premier fil (30a, 30c, 30e).

8. Cathéter (1a, 1b) selon la revendication 7, dans lequel
le coude s'étendant de manière hélicoïdale forme une spire d'au moins 180°.

9. Cathéter (1a, 1b) selon la revendication 7, dans lequel
le coude s'étendant de manière hélicoïdale forme une spire d'au moins 360°.

10. Cathéter (1b) selon la revendication 8 ou 9, dans lequel
un angle d'hélice du coude s'étendant de manière hélicoïdale diminue vers l'extrémité distale (11g).

11. Cathéter (1b) selon l'une quelconque des revendications 8 à 10, dans lequel
l'arc s'étend de façon elliptique.
